# EUROPÄISCHE PATENTSCHRIFT

(11) **EP 0 465 514 B1**
(45) Veröffentlichungstag und Bekanntmachung des Hinweises auf die Patenterteilung: **13.01.1993**
(21) Anmeldenummer: 90905181.5
(22) Anmeldetag: 05.04.1990
(51) Int. Cl.: A61F 2/44

(54) **Künstliche Bandscheibe**
Artificial spinal disc
Disque intervertebral artificiel

(30) Priorität: 08.04.1989 DE 3911610
(43) Veröffentlichungstag der Anmeldung: 15.01.1992
(73) Patentinhaber: ROBERT BOSCH GMBH, 70442 Stuttgart (DE)
(72) Erfinder: GOHL, Walter, D-7031 Aidlingen (DE); ESPER, Friedrich, D-7250 Leonberg (DE); OBERLE, Jürgen, D-7032 Sindelfingen (DE); HARMS, Jürgen, D-7517 Waldbronn (DE)
(86) Internationale Anmeldenummer: DE9000266
(87) Internationale Veröffentlichungsnummer: WO9011740

(56) Entgegenhaltungen:
- EP-A- 0 356 112
- EP-A-01 074 76
- EP-A-02 982 33
- US-A-43 097 77

## Beschreibung

Die Erfindung betrifft eine künstliche Bandscheibe zum Implantieren zwischen zwei Wirbeln einer Wirbelsäule.

Künstliche Bandscheiben müssen folgende Bedingungen erfüllen:
- als Dauerimplantat verwendbar sein,
- die in der Wirbelsäule auftretenden Stoßkräfte abfedern, damit die Wirbel nicht überlastet werden,
- die natürliche Wirbelbeweglichkeit möglichst wenig behindern,
- mit den benachbarten Wirbeln eine schub- und verdrehfeste Verbindung ermöglichen,
- unter den natürlichen Kipp- und Drehbelastungen keine Verschleißpartikel, noch sonstige Absonderungen an das umliegende Körpergewebe abgeben.

Bekannt ist beispielsweise eine künstliche Bandscheibe, welche aus einzelnen Teilen lose zusammengesetzt ist (Stern-Heft-Nr.36/86 vom 28.8.1986 S.102 ff). Dabei befindet sich zwischen zwei Metallplatten mit eingefügten Polyäthengleitstücken ein Gleitkern ebenfalls aus Polyäthylen. Die Befestigung dieser Prothesen zwischen zwei Wirbeln geschieht mittels kleiner Zähnchen, die sich auf der dem Knochen zugewandten Seite der Metallplatten befinden. Nachteilig hat sich hier u. a. folgendes erwiesen:
- nicht ausreichende Dauerfestigkeit des PE-Gleitkerns, insbesondere wegen seiner Alterungsanfälligkeit im Körpermedium;
- Kriechneigung des PE-Kerns;
- mangelnde Abriebbeständigkeit von PE-Gleitstücken;
- die schlechte Anpassung der Deckplatten an die Wirbeloberfläche;
- Knochenverletzungen durch die Zähnchen der Deckplatten.

### Vorteile der Erfindung

Eine Erfindung gemäß dem Hauptanspruch 1 besteht dagegen aus einer biokompatiblen Tragschicht, welche beidseits mit Deckplatten ebenfalls aus einem biokompatiblen Werkstoff fest belegt ist. Bevorzugt ist für die Tragschicht ein Silikongummi vorgesehen, da dieser Silikongummi sowohl die geforderte Biokompatibilität als auch die geforderte Stoßdämpfung aufweist. Als Werkstoff für die Deckplatten bietet sich ein kohlenstoffaser-verstärkter, biokompatibler Duroplast, z. B. ein Resiform-TCF (Triazinharz/C-Faser) an, wobei diese Platten fest mit dem Silikongummi verbunden werden. Hierdurch ist eine schub- und druckfeste Verbindung geschaffen, wobei die Silikongummischicht auch die notwendige Kippbewegung zuläßt.

Die Deckplatten sind knochenseitig so geformt, daß sie der Knochenkontur des Wirbelkörpers angepaßt sind. Ein zusätzlich vorgesehener Randwulst bewirkt die erforderliche Verdrehsicherheit.

Diese erfindungsgemäße künstliche Bandscheibe ist als Dauerimplantat geeignet. Zum schnelleren und stabileren Verbinden der TCF-Deckplatten mit den jeweiligen Wirbelkörpern wird noch eine Beschichtung dieser Deckplatten knochenseitig vorgenommen. Diese Beschichtung kann aus einer Hydroxylapatit- bzw. einer bestimmten Hydroxylapatit / Tricalciumphosphat-Mischung bestehen (HA/αTCP; HA/α-β-TCP).

Diese erfindungsgemäße Bandscheibe ist ein Verbundbauteil, welches als ganzes zwischen zwei Wirbeln eingesetzt werden kann und nicht erst bei der Implantation wie bei der bekannten künstlichen Bandscheibe aus zwei oder mehr Einzelteilen zusammengesetzt werden muß. In der Bandscheibe selbst entsteht bei Kippung keine Gleitreibung, d. h. es entsteht auch kein Abrieb, welcher sich bei Dauereinsatz im umgebenden Gewebe äußerst schädlich auswirkt.

Die Bandscheibe ist aus körperverträglichen, im Körper dauerhaltbaren Stoffen aufgebaut. Ferner sind die Deckplatten dieser Bandscheibe fest mit sich neu bildenden knöchernen Gewebe der angrenzenden Wirbel verbunden. Die Implantation ist ohne wesentliche Knochensektion an den Wirbelkörpern möglich.

Bevorzugt ist der Silikongummi vernetzt und an den z. B. aus Resiform-TCF bestehenden Deckplatten fest verbunden. Eine wesentliche Kriechdeformation und damit eine Abnahme der Kippbeweglichkeit tritt nicht auf.

### Zeichnung

Weitere Vorteile, Merkmale und Einzelheiten der Erfindung ergeben sich aus der nachfolgenden Beschreibung bevorzugter Ausführungsbeispiele sowie anhand der Zeichnung; diese zeigt in
Figur 1 eine Draufsicht auf eine erfindungsgemäße künstliche Bandscheibe;
Figur 2 einen querschnitt durch die künstliche Bandscheibe entsprechend Linie II-II in Figur 1;
Figur 3 einen querschnitt durch die künstliche Bandscheibe entsprechend Linie II-II in Figur 1 in einer weiteren Ausführungsform.

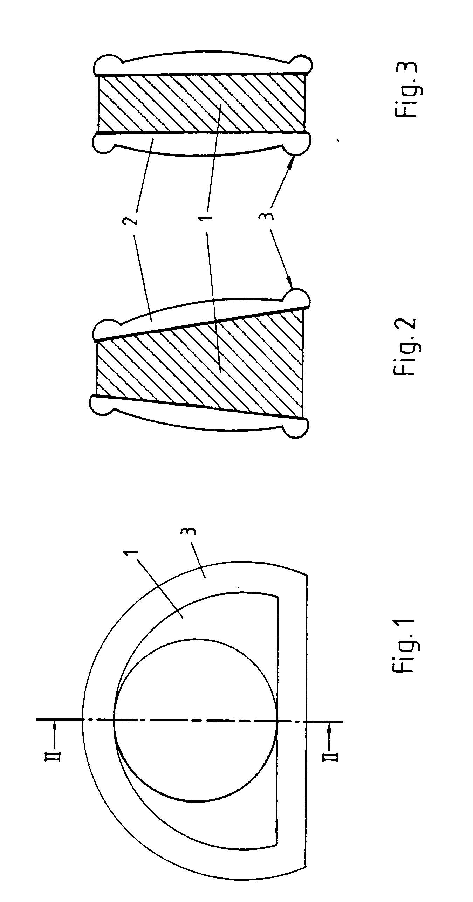

Eine erfindungsgemäße Bandscheibe entsprechend Figur 1 besteht aus einer Tragschicht 1 aus Silikongummi. Diese Tragschicht 1 kann, wie in Figur 3 gezeigt, je nach Anwendungsbedarf querschnittlich quaderförmig oder, wie in Figur 2 gezeigt, keilförmig zugeschnitten sein.

Erfindungsgemäß soll diese Tragschicht 1 beidseits durch Deckplatten 2 aus einem kohlenstoffaser-verstärkten Duroplast, vorzugsweise aus Resiform-TCF (Triazinharz / C-Faser) belegt sein. Dabei geht die Tragschicht 1 aus Silikongummi mit den Deckplatten 2 aus dem kohlenstoffaser-verstärkten Duroplast-Werkstoff eine feste Verbindung ein.

Die Tragschicht 1 aus Silikongummi bewirkt die geforderte Stoßdämpfung und verbindet die Deckplatten 2 schubfest miteinander, so daß auch eine notwendige Kippbewegung erlaubt ist

Die Deckplatten 2 sind knochenseitig so geformt, daß sie der Knochenkontur weitgehend angepaßt sind. Ein umlaufender Randwulst 3 bewirkt eine notwendige Verdrehsicherung.

Zum Verbinden der faserverstärkten Duroplast-Deckplatten mit dem Knochen werden die Deckplatten knochenseitig mit Hydroxylapatit (HA) bzw. einer bestimmten HA/α-TCP-bzw. HA/β-TCP-Mischung (Hydroxylapatit/Tricalciumphosphat) beschichtet.

## Patentansprüche

1. Künstliche Bandscheibe zum Implantieren zwischen zwei Wirbeln der Wirbelsäule, dadurch gekennzeichnet, daß eine Tragschicht (1) aus einem biokompatiblen Silikongummi beidseits mit Deckplatten (2) aus einem kohlenstoffaser-verstärkten, biokompatiblen Duroplast belegt ist.

2. Bandscheibe nach Anspruch 1, dadurch gekennzeichnet, daß die Deckplatten (2) fest mit der Tragschicht (1) verbunden sind.

3. Bandscheibe nach Anspruch 1 oder 2, dadurch gekennzeichnet, daß die Deckplatten (2) mit einem Randwulst (3) versehen sind.

4. Bandscheibe nach wenigstens einem der Ansprüche 1 bis 3, dadurch gekennzeichnet, daß die Deckplatten (2) mit Hydroxylapatit bzw. einer Hydroxylapatit/Tricalciumphosphat-β-Phase-Mischung (HA/α TCP; HA/α-β TCP) beschichtet sind.

## Claims

1. Artificial intervertebral disc for implanting between two vertebrae of the spinal column, characterised in that a substrate (1) of a biocompatible silicone rubber is covered on both sides by cover panels (2) of a carbon fibre-reinforced, biocompatible thermoset material.

2. Intervertebral disc according to Claim 1, characterised in that the cover panels (2) are firmly bonded to the substrate (1).

3. Intervertebral disc according to Claim 1 or 2, characterised in that the cover panels (2) are provided with an edge bead (3).

4. Intervertebral disc according to at least one of Claims 1 to 3, characterised in that the cover panels (2) are coated with hydroxylapatite or a hydroylapatite/tricalciumphosphate β-phase mixture (HA/αTCP; HA/α-β-TCP).

## Revendications

1. Disque intervertébral artificiel destiné à être implanté entre deux vertèbres de la colonne vertébrale, caractérisé en ce qu'une couche porteuse (1) en un caoutchouc de silicone biocompatible est revêtue des deux côtés de plaques de recouvrement (2) en résine thermodurcissable biocompatible renforcée par des fibres de carbone.

2. Disque intervertébral selon la revendication 1, caractérisé en ce que les plaques de recouvrement (2) sont fermement reliées à la couche porteuse (1).

3. Disque intervertébral selon la revendication 1 ou la revendication 2, caractérisé en ce que les plaques de recouvrement (2) sont munies d'un bourrelet de bordure (3).

4. Disque intervertébral selon au moins une des revendications 1 à 3, caractérisé en ce que les plaques de recouvrement (2) sont enduites d'un mélange phase β d'hydroxylapatite/tricalciumphosphate (HA/αTCP; HA/a - β TCP).
